# EUROPEAN PATENT APPLICATION

(11) **EP 2 664 275 A2**
(43) Date of publication of application: **20.11.2013**
(21) Application number: 13168083.7
(22) Date of filing: 16.05.2013
(51) Int. Cl.: A61B 5/053, A61M 25/09, A61B 19/00

(54) **Guide wire with position sensing electrodes for localization via measurement of bioimpedance**

(30) Priority: 17.05.2012 US 201213473962
(71) Applicant: Biosense Webster (Israel), Ltd., Yokneam 20692 (IL)
(72) Inventor: Govari, Assaf, 34400 Haifa (IL); Altmann, Andres Claudio, 34757 Haifa (IL); Beeckler, Christopher Thomas, Brea, CA California 92821 (US)
(74) Representative: Carpmaels & Ransford LLP

(57) **Abstract**

An apparatus includes a guide wire (22) configured to be inserted into a passage of a living body (28), and at least one electrical conductor (34) and electrical insulation covering a surface of the conductor (34) with a gap formed in the electrical insulation at a predefined position on the guide wire. A processor (46) is configured to measure an electrical impedance of the body (28) between the conductor (34) at the gap and one or more electrodes (R1,R2,R3) fixed to the body, and to calculate a location of the gap within the passage in the body in response to the measured impedance.

## Description

### FIELD OF THE INVENTION

The present invention relates generally to guide wires, and particularly to guide wire position tracking using bioimpedance measurements.

### BACKGROUND OF THE INVENTION

Guide wires are used in invasive medical therapies where an intra-body probe is percutaneously inserted into a body passage, such as a blood vessel from which different biological metrics are measured, or different therapies are applied via the intra-body probe. Common intra-body probes comprise catheters and balloon pumps.

### SUMMARY OF THE INVENTION

An embodiment of the present invention provides an apparatus including a guide wire and a processor. The guide wire is configured to be inserted into a passage of a living body, and includes at least one electrical conductor and electrical insulation covering a surface of the conductor with a gap formed in the electrical insulation at a predefined position on the guide wire. The processor is configured to measure an electrical impedance of the body between the conductor at the gap and one or more electrodes fixed to the body, and to calculate a location of the gap within the passage in the body in response to the measured impedance.

In some embodiments, the at least one electrical conductor includes multiple braided insulated conductor wires that form the guide wire, with respective gaps in the electrical insulation at predefined positions on the guide wire. In other embodiments, the at least one electrical conductor includes multiple twisted insulated conductor wires that form the guide wire, with respective gaps in the electrical insulation at predefined positions on the guide wire. Yet in other embodiments, the at least one electrical conductor includes multiple coiled insulated conductor wires that form the guide wire, with respective gaps in the electrical insulation at predefined positions on the guide wire. In some embodiments, the apparatus can also include at least one electrode, which is connected to the electrical conductor at the gap.

There is also provided, in accordance with an embodiment of the present invention, a method including inserting into a passage of a living body a guide wire, which includes at least one electrical conductor and electrical insulation covering a surface of the conductor with a gap formed in the electrical insulation at a predefined position on the guide wire. An electrical impedance of the body between the electrical conductor at the gap and one or more electrodes fixed to the body is measured. A location of the gap within the passage in the body is calculated in response to the measured impedance.

There is also provided, in accordance with an embodiment of the present invention, a method for producing a guide wire including providing at least one electrical conductor. A surface of the conductor is covered with electrical insulation. A gap in the electrical insulation is formed at a predefined position on the guide wire.

In some embodiments, providing the at least one electrical conductor includes providing multiple electrical conductors, covering the surface includes covering respective surfaces of the electrical conductors with the electrical insulation, forming the gap includes forming multiple gaps at predefined positions in the electrical insulation of the respective electrical conductors, and the method includes braiding, coiling, or twisting the multiple electrical conductors to form the guide wire.

The present invention will be more fully understood from the following detailed description of the embodiments thereof, taken together with the drawings in which:

### BRIEF DESCRIPTION OF THE DRAWINGS

Fig. 1 is a block diagram that schematically illustrates a system for tracking a guide wire using bioimpedance measurements, in accordance with an embodiment of the present invention; and
Fig. 2 is a flow chart that schematically illustrates a method for tracking a guide wire using bioimpedance measurements, in accordance with an embodiment of the present invention.

### DETAILED DESCRIPTION OF EMBODIMENTS

### OVERVIEW

During therapeutic techniques wherein intra-body probes are percutaneously inserted into body cavities such as a blood vessel, guide wires are often inserted to assist in navigating the intra-body probe throughout the desired body passage within the anatomical structure of interest. A conventional guide wire may comprise, for example, a mesh of interwoven exposed metal conductors, which are braided or twisted, and yet flexible and resilient.

Conventional real time tracking of the location of the guide wire within the passage of the patient's body typically involves using fluoroscopy or other radiative imaging techniques, which exposes the patient to unwanted radiation. The patient can also be placed in a magnetic field and magnetic position sensors can be attached to the guide wires for tracking purposes, but the narrow gauge of the guide wires makes this sort of approach technically difficult and costly.

Embodiments of the present invention that are described herein provide improved guide wire configurations, as well as methods and systems for tracking guide wires. In these embodiments, bioimpedance measurement techniques are utilized in tracking the position of a guide wire within the passage of a patient's body. The guide wire comprises insulated metal conducting wires that are braided or twisted, but each individual wire intentionally comprises an exposed region in the insulation that functions as an electrode. Within the braided guide wire, multiple such electrodes can be placed at different distances from the distal tip. Bioimpedance measurements are performed between the guide wire electrodes and fixed reference electrodes placed on the patient's body. The positions of the electrodes and thus the position of the guide wire within the passage of the patient's body are calculated in real time from the impedance measurements, and provided to the operator of the percutaneous therapy procedure.

The disclosed guide wires and associated methods provide the operator with an accurate, real time position of the guide wire in the patient body. Since the disclosed techniques are based on bioimpedance measurements, they do not expose the patient to harmful radiation. Moreover, bioimpedance electrodes can be integrated in a straightforward manner in known braided guide wire configurations. The electrodes have little or no effect on the cost and size of the guide wire.

### SYSTEM DESCRIPTION

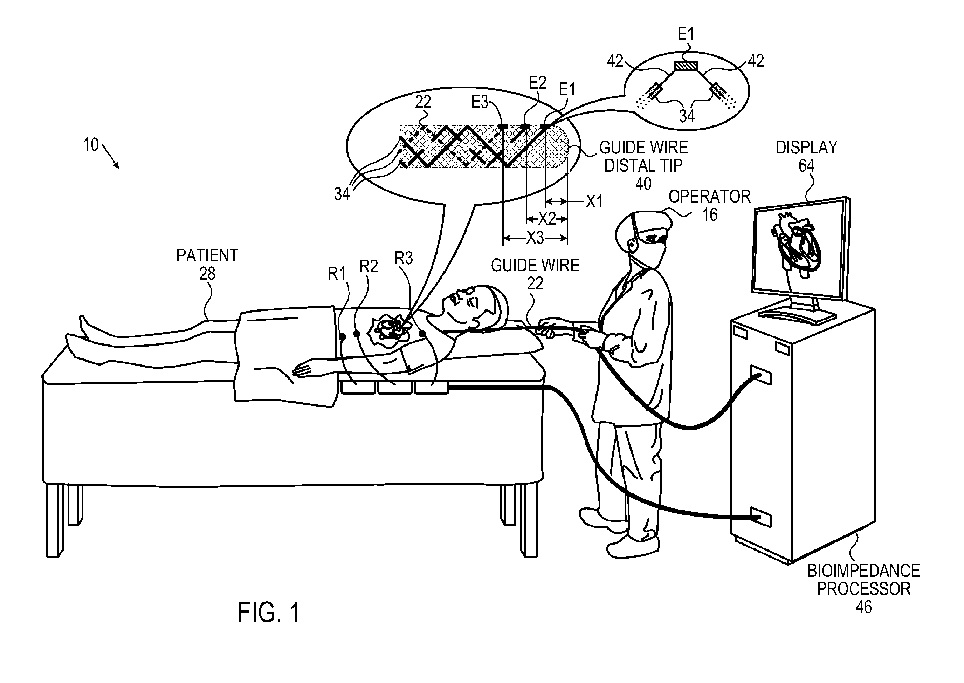

Fig. 1 is a block diagram that schematically illustrates the structure of a guide wire 22 inserted into a patient 28 using a guide wire bioimpedance measurement and tracking system 10, in accordance with an embodiment of the present invention. An operator 16 of system 10 percutaneously inserts guide wire 22 into patient 28. There are three reference electrodes R1, R2, and R3 fixed on the body of patient 28.

Guide wire 22 comprises insulated conductor wires 34, which are braided, coiled or twisted to form the guide wire. Three guide wire electrodes denoted E1, E2, and E3 near to a distal tip 40 of the guide wire are formed by gap openings 42 in the braided insulated wires which locally exposes the conductor as shown in the inset of Fig. 1. One respective electrode is fitted in each conductor wire 34. The centers of the three electrodes are located at distances X1, X2, and X3 from distal tip 40. (In the present example all three electrodes are shown in proximity to the distal tip, for the sake of clarity. Generally, the electrodes can be fitted at any desired distance from the distal tip, so as to enable tracking of any desired section of the guide wire.) In some embodiments as shown in Fig. 1, metal pads can be formed in and around the gap to improve the electrical contact between the exposed conductor at gap openings 42 of electrodes E1, E2, and E3 at distances X1, X2 and X3 from the distal tip of the guide wire, and the patient's body material.

In some embodiments, system 10 comprises a bioimpedance processor 46, which measures the electrical impedance between electrodes E1, E2 and E3 and reference electrodes R1, R2 and R3. Based on the measured impedances, processor 46 displays the location of guide wire 22 within the patient body on a display 64. In the embodiment shown in Fig. 1, the electrodes E1, E2, and E3, are connected through the guide wire to processor 46. The signal from reference electrodes R1, R2, and R3 are also connected to processor 46 as shown in Fig. 1. Electrical impedance measurements are made by the bioimpedance processor between the guide wire electrodes E1, E2, and E3 and reference electrodes R1, R2, and R3 in any combination between them.

Example methods related to impedance based measurements and position tracking of intra-body probes utilizing impedance based measurements are described in U.S. Patents 7,848,789, 7,848,787, 7,756,576, 7,684,850, and 7,536,218, which are incorporated herein by reference. The position of intra-body probes within a body passage of a patient relative to reference electrodes fixed on the patient's body, is determined by impedance measurements as further described within these references. Processor 46 may use any of these methods, or any other suitable method.

In some embodiments of the present invention, mapping the present position of guide wire 22 in the body of patient 28 uses calibration data of the impedances at different points along the body of patient 28. This data is typically stored in Bioimpedance Processor 46. The positions of the electrodes X1, X2, and X3 within the body passage are calculated from the measured impedances and the bioimpedance calibration data that was stored in processor 46. This guide wire position data is then mapped by processor 46 to display 64 along with an image of the desired passage of the patient's body from which operator 16 can track the motion of distal tip 40 of guide wire 22 in real time on display 64.

To further provide conceptual clarity of the embodiments of the present invention, and not by way of limitation whatsoever, the three distinct dashed/dotted lines representing three different and separate insulated conducting wires which are shown in the enlarged view of guide wire 22 in the inset of Fig. 1, are electrically isolated and configured to connect electrodes E1, E2, and E3 at distal tip 40 to processor 46. In the embodiments of the present invention, multiple such insulated conducting wires 34 are braided together in a mesh to form the guide wire 22. In other embodiments, multiple such insulated conducting wires are twisted together to form the guide wire. Yet in other embodiments, multiple such conducting wires are coiled together to form the guide wire.

The configuration of system 10 and of guide wire 22 are shown in Fig. 1 by way of conceptual clarity, and not be way of limitation whatsoever in the embodiments of the present invention. In alternative embodiments, any other suitable system and/or guide wire configuration can be used. For example, the guide wire can comprise any desired number, N, of electrodes E1, E2, ...EN formed by gaps in the insulation at distances X1, X2, ...XN relative to the distal tip of the guide wire, or any other appropriate reference position. In an embodiment, N=1, i.e., the guide wire comprises a single conductor with a single electrode. System 10 may comprise any desired number, M, of reference electrodes R1, R2, ...RM fixed to the body of the patient. In the embodiments of the present invention, processor 46 may perform impedance measurements between the N guide wire electrodes and the M fixed reference electrodes.

Some elements of processor 46 may be implemented in hardware, e.g., in one or more Application-Specific Integrated Circuits (ASICs) or Field-Programmable Gate Arrays (FPGAs). Additionally or alternatively, some processor elements can be implemented using software, or using a combination of hardware and software elements. In some embodiments, processor 46 comprises a general-purpose computer, which is programmed in software to carry out the functions described herein. The software may be downloaded to the computer in electronic form, over a network, for example, or it may, alternatively or additionally, be provided and/or stored on non-transitory tangible media, such as magnetic, optical, or electronic memory.

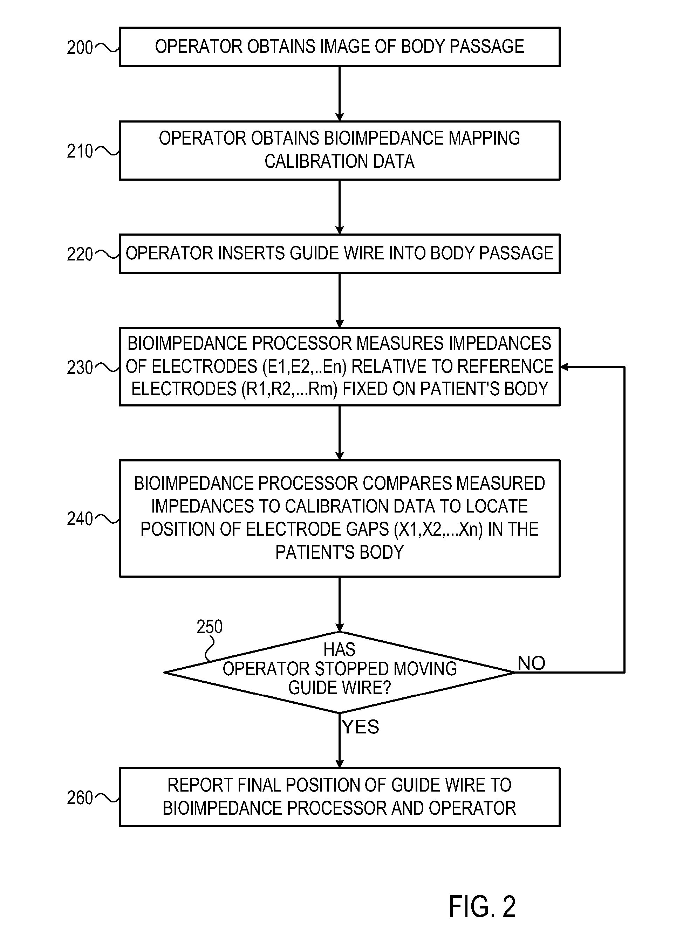

Fig. 2 is a flow chart that schematically illustrates the steps in determining the positions of electrodes on a guide wire in a body passage using bioimpedance measurements, in accordance with an embodiment of the present invention.

In an imaging step 200, operator 16 obtains an image of the body passage, which is stored in the bioimpedance processor. In a calibration step 210, the operator obtains bioimpedance calibration data, which typically comprises a mapping of the impedance along the body passage onto the image of the body passage from imaging step 200, and is stored in bioimpedance processor 46.

In an insertion step 220, the operator inserts guide wire 22 into a body passage, such as a blood vessel. In an impedance measurement step 230, the bioimpedance processor measures impedances of electrodes (E1, E2, ...EN) relative to reference electrodes (R1, R2, ...RM) fixed on the patient's body. In a comparison step 240, the bioimpedance processor compares the measured impedance to the calibration data from calibration step 210 to locate the position of the electrode gaps (X1, X2, ...XN) in the patient's body.

In an evaluation step 250, if operator 16 stopped moving guide wire 22, the final position of the guide wire in the body passage is reported to bioimpedance processor 46 and to operator 16 in a reporting step 260. The operator can view the position of the guide wire on display 64 mapped onto an image of the body passage, such as a blood vessel or heart. If operator 16 has not stopped moving the guide wire in the body passage, the bioimpedance controller continues to measure the impedance in impedance measurement step 230.

Although the embodiments described herein mainly address guide wire electrodes for bioimpedance position sensing in human patients, the methods and systems described herein can also be used in other applications, whereby insulated conducting wires, filaments, or any other conducting structures of the like, comprising insulation gaps or insulation openings which are configured to form separate, electrically isolated electrodes on the surface of an intra-body probe. These electrodes can be configured to detect biological signals or apply signals used in a variety of invasive percutaneous medical therapies to any living body.

It will thus be appreciated that the embodiments described above are cited by way of example, and that the present invention is not limited to what has been particularly shown and described hereinabove. Rather, the scope of the present invention includes both combinations and sub-combinations of the various features described hereinabove, as well as variations and modifications thereof which would occur to persons skilled in the art upon reading the foregoing description and which are not disclosed in the prior art. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

## Claims

1. An apparatus, comprising:
a guide wire, which is configured to be inserted into a passage of a living body, and which comprises at least one electrical conductor and electrical insulation covering a surface of the conductor with a gap formed in the electrical insulation at a predefined position on the guide wire; and
a processor, which is configured to measure an electrical impedance of the body between the conductor at the gap and one or more electrodes fixed to the body, and to calculate a location of the gap within the passage in the body in response to the measured impedance.

2. The apparatus according to claim 1, wherein the at least one electrical conductor comprises multiple braided insulated conductor wires that form the guide wire, with respective gaps in the electrical insulation at predefined positions on the guide wire.

3. The apparatus according to claim 1, wherein the at least one electrical conductor comprises multiple twisted insulated conductor wires that form the guide wire, with respective gaps in the electrical insulation at predefined positions on the guide wire.

4. The apparatus according to claim 1, wherein the at least one electrical conductor comprises multiple coiled insulated conductor wires that form the guide wire, with respective gaps in the electrical insulation at predefined positions on the guide wire.

5. The apparatus according to claim 1, and comprising at least one electrode, which is connected to the electrical conductor at the gap.

6. A method, comprising:
inserting into a passage of a living body a guide wire, which comprises at least one electrical conductor and electrical insulation covering a surface of the conductor with a gap formed in the electrical insulation at a predefined position on the guide wire;
measuring an electrical impedance of the body between the electrical conductor at the gap and one or more electrodes fixed to the body; and
calculating a location of the gap within the passage in the body in response to the measured impedance.

7. The method according to claim 6, wherein the at least one electrical conductor comprises multiple braided insulated conductor wires that form the guide wire, with respective gaps in the electrical insulation at predefined positions on the guide wire.

8. The method according to claim 6, wherein the at least one electrical conductor comprises multiple twisted insulated conductor wires that form the guide wire, with respective gaps in the electrical insulation at predefined positions on the guide wire.

9. The method according to claim 6, wherein the at least one electrical conductor comprises multiple coiled insulated conductor wires that form the guide wire, with respective gaps in the electrical insulation at predefined positions on the guide wire.

10. A method for producing a guide wire, the method comprising:
providing at least one electrical conductor;
covering a surface of the conductor with electrical insulation; and
forming a gap in the electrical insulation at a predefined position on the guide wire.

11. The method according to claim 10, wherein providing the at least one electrical conductor comprises providing multiple electrical conductors, wherein covering the surface comprises covering respective surfaces of the electrical conductors with the electrical insulation, wherein forming the gap comprises forming multiple gaps at predefined positions in the electrical insulation of the respective electrical conductors, and comprising braiding, coiling, or twisting the multiple electrical conductors to form the guide wire.
